Europäisches Patentamt

European Patent Office

Office européen des brevets

(11) Publication number: **0 483 765 A2**

# EUROPEAN PATENT APPLICATION

(21) Application number: **91118439.8**

(22) Date of filing: **29.10.91**

(51) Int. Cl.5: **A61K 35/14**, A61M 1/36

(30) Priority: **01.11.90 JP 296850/90**

(43) Date of publication of application:
**06.05.92 Bulletin 92/19**

(84) Designated Contracting States:
**DE FR GB IT**

(71) Applicant: **JAPAN IMMUNORESEARCH LABORATORIES CO., LTD.**
**351-1, Nishiyokote-cho**
**Takasaki-shi, Gunma(JP)**

(72) Inventor: **Adachi, Masakazu**
**3493-9, Ishiharamachi**
**Takasaki-shi, Gunma(JP)**

(74) Representative: **Wächtershäuser, Günter, Dr.**
**Tal 29**
**W-8000 München 2(DE)**

(54) **Method for activating lymphocyte.**

(57) The present invention provides a method and an apparatus therefor to promote sufficient expression of the lymphocyte activating potential which cytokines such as IL-2 have essentially. The method comprises contacting blood from a tumor patient with a carrier of a higher affinity to granulocyte than to lymphocyte, thereby removing granulocyte from the blood, subsequently adding cytokine to the blood in the state where the ratio of granulocyte to lymphocyte is decreased, and recirculating the blood into the body of the tumor patient, whereby is obtained an excellent effect on the suppression of tumor proliferation.

## BACKGROUND OF THE INVENTION

### 1) Field of the Invention

The present invention relates to a method for activating lymphocyte and an apparatus to be used therefor. More specifically, the present invention relates to a method for activating lymphocyte and an apparatus to be used therefor, a method comprising removing granulocyte from blood and adding cytokine to the blood in the state where the ratio of granulocyte to lymphocyte is decreased, whereby the lymphocyte in the blood is activated.

### 2) Description of the Related Art

The research works of cancer therapy and immunology have made remarkable progress in 1980's, since attention has been focused on cytokine in the field of immunology. It has been elucidated that lymphocyte, monocyte and macrophage, stimulated with cytokine, generate a wide variety of protein activating factors having biological activities, and that immune response is adjusted and modified via the factors. Additionally, a great number of recent clinical trials have demonstrated the relationship between the disease symptom and the immune system of cancer patients. Conventionally, the protein factor generated by monocyte and macrophage has been referred to as monokine, while the protein factor principally generated by lymphocyte has been referred to as lymphokine. It has been found recently that various protein factors are generated by various types of cells. They are now referred to as cytokine in general, and a number of findings thereof are reported: cytokine is not generated by a certain type of cell, but by various types of cells; the cytokine generating cells generally do not generate cytokine in constant manner, but the cells initiate to generate cytokine on stimulation; an identical type of cells generate plural types of cytokine; the actions thereof have not only local effects but also systemic effects; and some of the actions are mutually in common. For example, all of interleukin-1 (IL-1), interleukin-2 (IL-2), interferon (IFN), and tissue necrosis factor (TNF) have anti-tumor activity and pyrogenicity, while IL-1, IL-2, IL-4, IL-6, IL-7, TNF and GM-CSF (granulocyte-macrophage colony stimulating factor) have actions to promote the mitosis of T cell. Various networks are formed between cytokine and cytokine generating cells or target cells. Via the networks are adjusted the generation and the activity of cytokine. Macrophage activated with TNF, for example, induces the generation of IL-1, IL-6, GM-CSF and the like, while macrophage activated with IL-4 suppresses the generation of IL-1 and TNF. IL-2 and IFN-r enhance the activity of LAK cell (Lymphokine-activated-killer cell), but IFN-$\alpha$, IFN-$\beta$, and IL-3 inhibit the activity.

As has been mentioned heretofore, cytokine has direct or indirect anti-tumor activity. Therefore, a great number of clinical trials have been carried out for the objective to use the cytokine for cancer therapy. However, no therapeutical effects of cytokine have observed as were expected initially. For example, IL-2 is one species of cytokine which was found, as a factor to selectively grow human T cell, in the supernatant of the culture of human peripheral leukocyte stimulated with PHA (phytohemagglutinin), by Morgan in 1976 (Morgan, D.A. et al., Science, 192, 1007 (1976)). IL-2 was firstly referred to as T-cell growth factor. The anti-tumor activity of IL-2 was regarded possibly due to the growth and enhanced activities of LAK cell, cytotoxic T cell (CTL), B cell, natural killer (NK) cell and the enhanced production of IFN-$\gamma$ (Tetsuo Taguchi, Cancer and Chemotherapy, 13, 1(1986)). Clinical trials were thus carried out. However, no single therapy of IL-2 could bring about the therapeutical effects as had been expected initially. On the contrary, there were observed a high frequency of adverse effects such as fever, chill and trepidation, and anorexia (Tetsuro Sano, Cancer and Chemotherapy, 14, 903(1986)). Rosenberg et al. also reported the combined therapy with IL-2 and IL-2 activated LAK cell (Rosenberg, S.A., et al., N. Engl. J. Med., 316, 889(1987)). Subsequently, a great number of research works have been devoted to the therapeutical method, but such research works have demonstrated the efficacy of the method only on some of cancers such as kidney cancer and melanoma. Adverse effects due to the therapeutical treatment, including fever and symptoms of gastrointestinal tract, occurred frequently. Costly expense of the therapeutical treatment along with the adverse effects could not produce the effects as expected initially (Rosenberg, S.A., et al., N. Engl. J. Med., 313, 1485-(1985); Tetsuo Taguchi, Novel Medicine, 42, 325(1987); Fisher, R.I., et al., Proceeding of ASCO., 6,246-(1987)). The combined therapy of IL-2 with other cytokines having anti-tumor activity cannot bring about a higher efficacy (Nagahiro Saijyo, Proceedings of Japanese Cancer Association, 46, 289(1987)).

As has been described above, it has been assumed as the potential reason of the anti-tumor effect of IL-2 lower than those as was expected, that the absolute number of LAK cells with respect to the number of tumor cells may be less, that LAK cells may not be accumulated in tumor, or that the activation of suppressive T cell and the formation of immune complex drives the tumor cells in organisms with tumor, into the state escaping from anti-tumor immune system. According to the report by Ogura et al., the localization of the LAK cells labeled with radioisotopes was not observed in the examination of the pharmacokinetics of LAK cell (Tsuyoshi Ogura, Internal Medicine, 66(4), 777(1990)). In order to elucidate these issues, various research works are now going on, namely the development of the culture method of IL-2 and the apparatus therefor, the investigation on the dose of IL-2 and the timing of IL-2 dose, and the combined therapy thereof with other agents and other types of cytokines. However, satisfactory outcome has not been obtained yet.

## SUMMARY OF THE INVENTION

The present inventors have made intensive investigations so as to develop the means to promote sufficient expression of the lymphocyte activating potential that cytokines such as IL-2 have essentially. Consequently, they have found that lymphocyte in blood can be efficiently activated, by removing granulocyte from blood and adding cytokine to the blood in the state where the ratio of granulocyte to lymphocyte is decreased. The ratio of granulocyte to lymphocyte is referred to as G/L ratio hereinafter. The blood with lymphocyte activated is then recirculated into the body of a patient, whereby is obtained an excellent effect on the suppression of tumor proliferation. Thus, they have achieved the present invention.

The present invention is to provide a method for activating lymphocyte in blood, comprising contacting blood with a carrier of a higher affinity to granulocyte than to lymphocyte, thereby removing granulocyte from the blood and subsequently adding cytokine to the blood in the state where the ratio of granulocyte to lymphocyte is decreased, whereby the lymphocyte in the blood is activated.

The present invention also provides an apparatus for activating lymphocyte in blood, characterized by having a granulocyte adsorbing part containing a carrier with a higher affinity to granulocyte than to lymphocyte, and a cytokine injection part for adding cytokine to the blood flown out of the adsorbing part.

## BRIEF DESCRIPTION OF THE DRAWINGS

Fig.1 is a view of one embodiment of the apparatus for activating lymphocyte of the present invention.

Fig.2 is a view representing the relation between G/L ratio and the prognosis of tumor patients.

Fig.3 is a view representing the relation between the number of exdgeneous circulation and the growth rate of tumors in Example 2.

## DESCRIPTION OF PREFERRED EMBODIMENTS

As the carrier with an affinity to granulocyte higher than the affinity to lymphocyte (simply referred to as "carrier" hereinafter) and the granulocyte adsorbing part containing the carrier to be used in the method and the apparatus for the present invention, those disclosed in Japanese Patent Laid-open No.193069/1990, for example, may be used. Any carrier non-toxic to blood in contact may be used with no relation to the material thereof. Preferably, a carrier with a contact angle to water being in the range of 55 to 95° is used. Specifically, polystyrene, cellulose acetate, nylon such as 6-Nylon and 11-Nylon, polytrifluoroethylene, polyethylene terephthalate are particularly preferable. Two or more of the carriers described above may be combined together. The form and size of the carriers are not specifically limited, but the contact efficiency to blood should be high. For example, a beads carrier of a diameter of approximately 0.1 to 10 mm may be employed.

The granulocyte adsorbing part will now be explained more specifically. About 4,000 cellulose acetate beads of a 2.0-mm particle size (manufactured by Sekisui Chemicals, Co. Ltd.) were immersed in 500 ml of a surfactant, 5% SCAT20X-N (manufactured by Daiichi Pharmaceutical Chemicals, Co. Ltd.) for 30 minutes, followed by washing in deionized water five times and subsequent washing in 99.5% ethanol two times, which were then dried in air and finally irradiated by $\gamma$ ray of the total radiation dose of 2.5 Mrad. A 50-ml column packed with the beads thus prepared is preferably employed. The blood volume in the column packed with the beads is preferably about 50 ml.

If blood is in contact with such carrier, the granulocyte in the blood is selectively adsorbed and removed to decrease the G/L ratio of the blood. As is described in Japanese Patent Laid-open No.193069/1990, the G/L ratio in the blood of tumor patients is higher than the ratio in the blood of healthy subjects, and has a good correlation with the prognosis of the patients. Hence, the reduction in the G/L ratio of the blood of tumor patients indicates better prognosis of the patients. However, it does not eliminate the

EP 0 483 765 A2

tumor per se. According to the present invention, it has been found that cytokine is added to the blood in which the G/L ratio is preliminarily reduced, whereby the lymphocyte in the blood is remarkably activated, with consequent results of excellent suppressive effects on tumor proliferation.

The cytokine to be added to the blood with a reduced G/L ratio includes, for example, IL-1$\alpha$, IL-1$\beta$, TNF-$\alpha$, TNF-$\beta$ and the like, considered to have anti-tumor activity; IL-4, IL-6 and IL-7, considered to have the action to promote the growth of T cell; and IL-5 and TGF (T-cell growth factor), having the action to promote the mitosis and proliferation of B cell, besides IL-2, IFN-$\alpha$, IFN-$\beta$ , IFN-$\gamma$ and the like. These types of cytokines may be any one of natural type and genetic recombinant type. IL-2 is specifically preferable among them. The IL-2 may be the supernatant of the IL-2 containing culture, already known and induced in vitro, and the purified sample thereof; and the IL-2 produced according to genetic recombination technique and the IL-2s having the same effects (rIL-2), for example a synthetic peptide of a part of the amino acid sequence of the natural IL-2, as is disclosed in Japanese Patent Laid-open No.246322/1985. The recombinant human IL-2 obtained by genetic recombinant technique is particularly preferable among them.

The apparatus for activating lymphocyte in accordance with the present invention will now be explained with reference to the drawings showing one embodiment of the present invention.

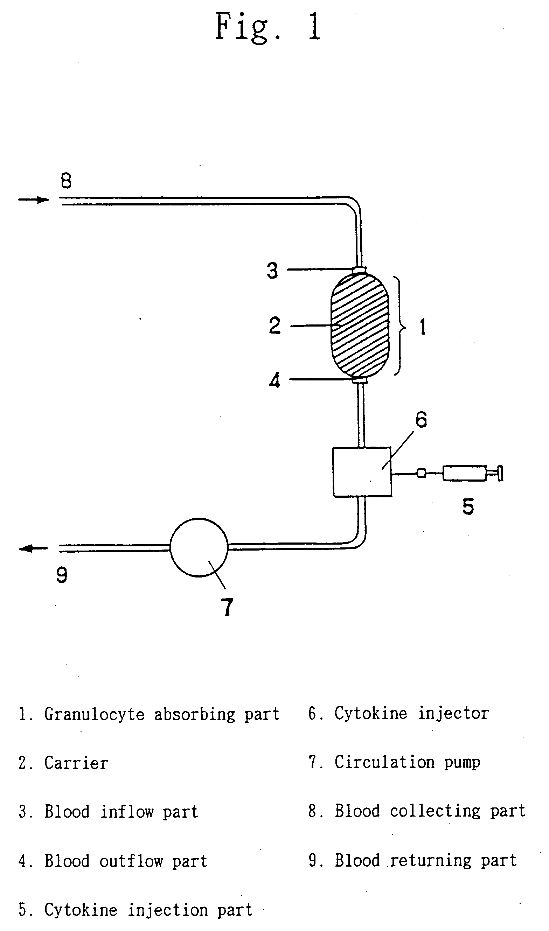

In Fig.1, 1 is the granulocyte adsorbing part described above, which is packed with glass beads carrier 2. One end of such granulocyte adsorbing part is equipped with a blood inflow part in order that blood (venous or artery blood from a patient) for treatment is flown out from blood collecting part 8 into the granulocyte adsorbing part, while the other end is equipped with blood outflow part 4, in order that the blood from which granulocyte is removed on contact with the carrier and which has a consequent reduced G/L ratio is flown out of the granulocyte adsorbing part. The apparatus of the present invention is furthermore equipped with cytokine injection part 6 having cytokine injection port 5 to add cytokine to the treated blood being flown out of blood outflow part 4 and having a reduced G/L. The apparatus is equipped with circulation pump 7 to recirculate the blood containing activated lymphocyte in the body of the patient. The blood is then made to return to blood returning part 9.

The apparatus of the present invention can be used so as to continuously remove granulocyte during exogeneous circulation to reduce the G/L ratio according to general plasma exchange therapy and so as to activate lymphocyte. The apparatus may be equipped with silicon rubber tube and non-toxic pipes including polyvinyl chloride as transferring materials. The apparatus may be equipped with a heating device to elevate the temperature of blood which is lowered during circulation, general detectors to examine the blood during the circulation, and a blood component making-up device to compensate the insufficiency of the blood components other than the component granulocyte, which is caused by the present invention. Component transfusion, for example, may be used for making up such insufficiency of the blood components.

The method of exogeneous circulation in rabbit comprises 5 to 20 cycles of 1 to 3 circulations per week, the circulation being carried out at a rate of 1 to 2 ml/min for 1 to 2 hours, followed by adding 1.0 ml of one type of cytokine, for example, IL-2, of a concentration adjusted to 10 U/ml to $1 \times 10^3$ U/ml, from injection part 6. Such exogeneous circulation carried out for 1 to 2 hours, is defined as one cool. By carrying out 2 to 20 cools described above, the activation of lymphocyte is satisfactorily effected. Thus, anti-tumor effects of the activated lymphocyte on tumor cells can be enhanced.

The method of exogeneous circulation in humans comprises 5 to 20 cycles of 1 to 3 circulations per week, the circulation being carried out at a rate of 50 to 150 ml/min for 1 to 2 hours, followed by the addition of 1.0 ml to 10.0 ml of one type of cytokine, for example, IL-2, of a concentration adjusted to 10 U /ml to $1 \times 10^5$ U/ml, from injection part 6. Such exogeneous circulation carried out for 1 to 2 hours, is defined as one cool. Two to 20 cools described above may be enough.

According to the present invention, the G/L ratio of the blood from tumor patients can be lowered, and the anti-tumor effects of activated lymphocyte on tumor cells can be enhanced by the following administration of cytokine to activate lymphocyte.

Example

The present invention will now be explained specifically in examples, but the present invention is not limited to these examples.

In the following examples, the number of granulocyte (G) and the number of lymphocyte (L) were measured with an automatic analyzer Sysmex E-4000 manufactured by Toa Medicinal Electronics, K.K. to calculate the G/L ratio, when not otherwise specified.

4

Reference Example 1

Prognosis of tumor patients and G/L ratio

Examination was carried out of the prognosis of the following four groups of 114 cases in total; A group consisting of 37 progressive tumor patients with a G/L ratio in the range of of 2.0 or less; B group consisting of 37 progressive tumor patients with a G/L ratio in the range of above 2.0 and 3.0 or less; C group consisting of 24 progressive tumor patients with a G/L ratio in the range of above 3.0 and 4.0 or less; and D group consisting of 16 progressive tumor patients with a G/L ratio in the range of above 4.0. The tumors as the subjects were, stomach cancer, breast cancer, lung cancer, colon cancer, cervical cancer, esophageal cancer and spleen cancer. No significant difference ($X^2$-test) was observed in any background factor of P.S. of 0 to 4.

The comparative results based on survival curves, according to Kaplan-Meier method, (see "Practical Statistics for Judgment of Therapeutic Efficacy", Hirotami Tominaga, Kani Shobo, issued August 20, 1982) are shown in Table 2.

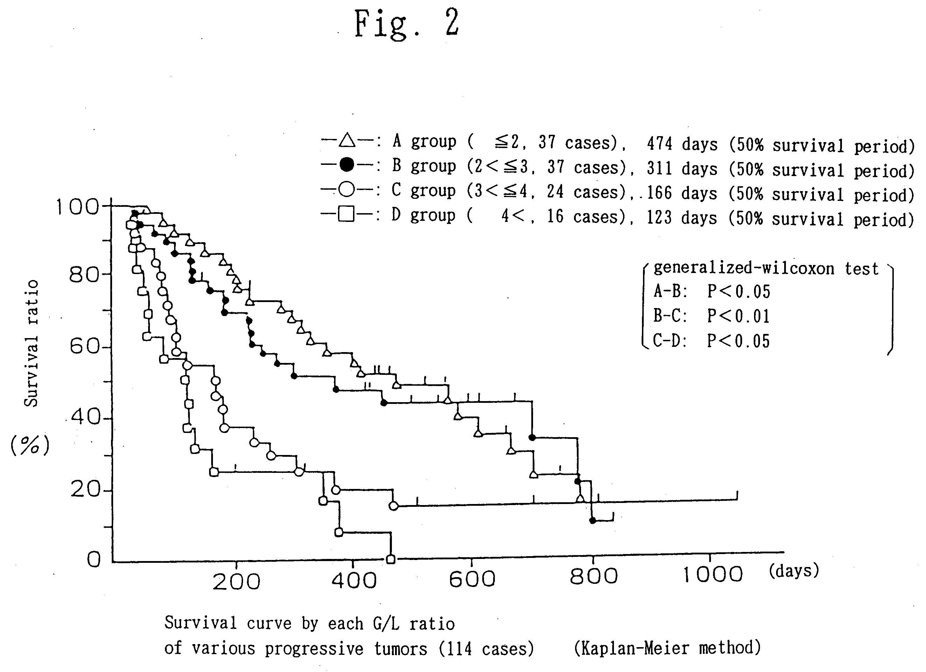

In Fig.2, survival rate in % is shown on axis of ordinates and the time after the treatment is shown in days on axis of abscissa.

Fig.2 shows, that the survival rate of A group is significantly higher than that of B group; the survival rate of B group is significantly higher than that of C group; the survival rate of C group is significantly higher than that of D group; and that the groups with higher survival rates have better prognosis. Generalized Wilcoxon Test was employed as the statistical method mentioned hereinabove. 50% survival period is the longest in A group, ie. 474 days. It is 311 days in B group; and it is 166 days in C group. The survival period is the shortest in D group, ie. 123 days.

Table 1 shows the tumor types of 114 cases with various progressive tumors, the number of each group included in a specified range of G/L ratios and the number of patients dead or in survival.

Table 1

The tumor types of 114 cases with various progressive tumors and the number of each group included in a specified range of G/L ratios

| Tumor types Survival | | Stomach | Breast | Lung | Colon | Cervical |
|---|---|---|---|---|---|---|
| G/L ratio A group <=2 (37 cases) | survive | 2 | 2 | 3 | 1 | 3 |
| | dead | 8 | 3 | 5 | 3 | 2 |
| | unknown | 0 | 0 | 0 | 0 | 0 |
| G/L ratio B group 2< <=3 (37 cases) | survive | 2 | 1 | 2 | 0 | 2 |
| | dead | 10 | 2 | 4 | 1 | 4 |
| | unknown | 1 | 1 | 0 | 3 | 0 |
| G/L ratio C group 3< <=4 (24 cases) | survive | 1 | 1 | 0 | 0 | 0 |
| | dead | 5 | 1 | 7 | 2 | 2 |
| | unknown | 0 | 0 | 0 | 0 | 1 |
| G/L ratio D group 4< (16 cases) | survive | 0 | 0 | 0 | 0 | 0 |
| | dead | 3 | 1 | 3 | 2 | 2 |
| | unknown | 0 | 0 | 0 | 0 | 1 |
| Total (1) | survive | 5 | 4 | 5 | 1 | 5 |
| | dead | 26 | 7 | 19 | 8 | 10 |
| | unknown | 1 | 1 | 0 | 3 | 2 |

| Esophageal | Spleen | Total (2) |
|:---:|:---:|:---:|
| 0 | 0 | 11 |
| 2 | 1 | 24 |
| 2 | 0 | 2 |
| 1 | 0 | 8 |
| 1 | 0 | 25 |
| 2 | 0 | 4 |
| 0 | 0 | 2 |
| 2 | 1 | 20 |
| 1 | 0 | 2 |
| 0 | 0 | 0 |
| 3 | 1 | 15 |
| 0 | 0 | 1 |
| 1 | 0 | 21 |
| 8 | 3 | 84 |
| 5 | 0 | 9 |
| | Total | 114 |

Example 1

Effects of lymphocyte activating method on rabbits with transplanted tumor

On the back of a female Japanese White rabbit weighed 3.0 kg (supplied from Experimental Animals, K.K.) was subcutaneously transplanted $1 \times 10^7$ of $VX_2$ tumor derived from Shope papilloma virus. After the confirmation of the success of tumor transplantation, the removal of granulocyte and administration of IL-2 via exogeneous circulation was initiated on day 16 from the transplantation. The exogeneous circulation at two times per week was carried out nine times in total. A surflow indwelling needle for ear vein, Type C 22G x 1/4, manufactured by TERUMO, Co. Ltd., was connected to an extension tube of X2-5, manufactured by Topp, K.K.. The needle was then inserted into the rabbit ear vein on one side, which was used as the blood collecting part. The blood was passed through a 50-ml column packed with about 4,000 cellulose acetate resin beads of a 2.0-mm particle size (manufactured by Sekisui Chemicals, Co. Ltd.) as granulocyte adsorbing beads to remove granulocyte. Circulation was effected at a flow of 1 ml/min. A pump of SJ-1211 manufactured by ATTO Co. Ltd., was used as the circulation pump for exogeneous circulation. The blood was made to return into the catheter tube inserted into the rabbit ear vein on the other side to prepare an exogeneous circulation route. Recombinant human IL-2 (rIL-2; manufactured by Genzyme Co. Ltd.) was employed as the IL-2, which was adjusted at the concentration of 100 U/ml. One hour later after the initiation of the exogeneous circulation, 1 ml of the adjusted recombinant human IL-2 was added to the blood during the exogeneous circulation, from the cytokine injection part on the blood outflow part of the column. The blood thus treated was then made to return into the body of the rabbit, followed by further circulation for one hour. Similar rabbits with transplanted tumor were left as they were, and on day 16 after the transplantation, they were administered with IL-2 prepared in the same manner, two times per week. Those rabbits were used as controls. The results described above are shown in Table 2.

The days after the transplantation in Table 2 show that the first and ninth exogeneous circulation were carried out on days 16 and 48, respectively. The test results according to the treatment of the present invention are shown on left side of the Table, representing the size of tumors after the removal of granulocyte via the exogeneous circulation followed by the administration of IL-2, as well as the G/L ratio at the outflow part of the column. With no treatment for granulocyte removal, IL-2 was administered to control rabbits with transplanted tumor, from ear vein. The test results of these control rabbits are shown on right side of the Table. The G/L ratio of the control rabbits were determined, by measuring the blood collected from the rabbit ear peripheral blood. The size of the tumors is represented by the area calculated by multiplying the short diameter by the long diameter of each tumor.

## Table 2

### Effects of IL-2 after the treatment of granulocyte removal

### via exogeneous circulation

| Days after transplantation (days with circulation treatment) | Group with treatment for granulocyte removal through exogeneous circulation and IL-2 dose | | Control group (IL-2 administered group) | |
|---|---|---|---|---|
| | G/L ratios at column outflow part | Size of tumor (Area; $mm^2$) | G/L ratios at column outflow part | Size of tumor (Area; $mm^2$) |
| 16 (1) | 0.50 | 414 | 1.08 | 100 |
| 18 (2) | 0.30 | 500 | 1.09 | 150 |
| 22 (3) | 0.21 | 572 | 0.65 | 621 |
| 25 (4) | 0.37 | 414 | 1.50 | 975 |
| 29 (5) | 0.36 | 280 | 1.46 | 1450 |
| 32 (6) | 0.29 | 143 | 2.10 | 2331 |
| 39 (7) | 0.22 | 100 | 2.59 | 3185 |
| 45 (8) | 0.28 | 25 | 3.27 | 4080 |
| 48 (9) | 0.27 | 0 | 3.40 | 5600 |

As is apparently shown in Table 2, the tumors were eliminated in the group to which was applied the method of the present invention, namely the group treated with the exogeneous circulation for the removal of granulocyte and with the dose of IL-2. The result suggests that the G/L ratio of the blood from which granulocyte was removed via the exogeneous circulation is remarkably decreased and that the lymphocyte activated with IL-2 has anti-tumor effects.

Since the rabbits which received the singly therapy of IL-2 dosage without the treatment of the removal of granulocyte had higher G/L ratios, the lymphocyte activated with IL-2 could not exhibit the anti-tumor effects sufficiently.

Example 2

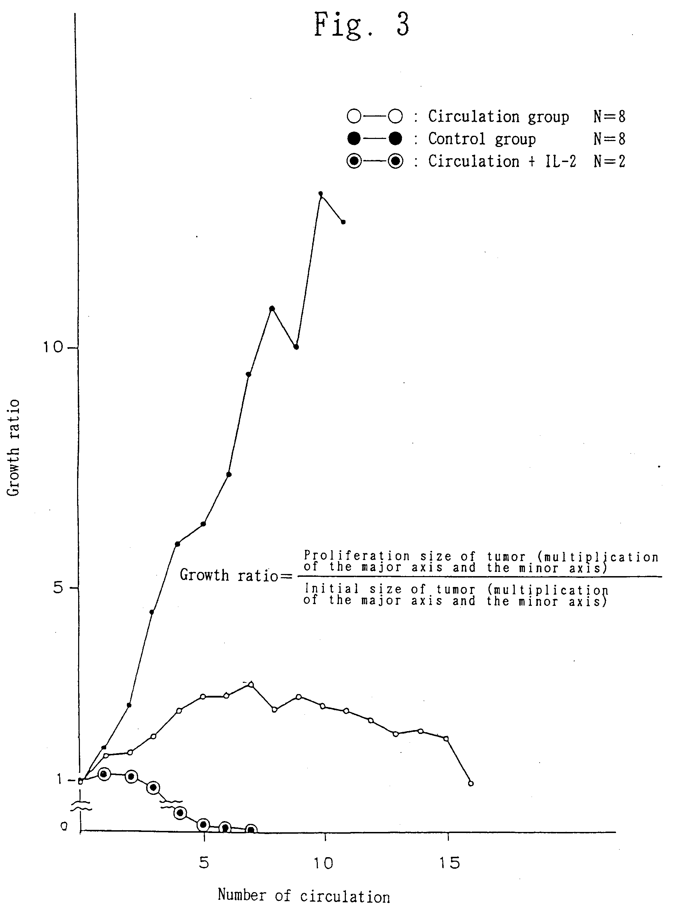

A group consisting of 8 rabbits without the exogeneous circulation by means of the same experimental system described in Example 1 and with no administration of IL-2, was used as a control group. A group consisting of 8 rabbits, treated only with the exogeneous circulation, was used as a circulation group. A group consisting of 2 rabbits treated with the exogeneous circulation to reduce the G/L ratio and subsequently administered with IL-2, was used as a (circulation + IL-2) group. Animals were divided in these three groups. The size of tumors was periodically examined and compared with the initial size of the tumors, namely the initial area calculated by multiplying a long diameter by a short diameter. The initial size was individually defined as 1. The results are shown in Fig.3.

In Fig.3, the axis of ordinates represents the growth ratio of tumors and the axis of abscissa represents the number of circulation.

As is apparently shown in Fig.3, the tumors in the control group proliferated over time and the rabbits with tumor growth were dead. Tumors temporarily proliferated in the circulation group, but the proliferation of tumors was inhibited as the number of circulation increased. The proliferation of tumors was inhibited, as the respective numbers of circulation and the dose of IL-2 increased in the (circulation + IL-2) group to which was applied the method of the present invention. Furthermore, the tumors were eliminated on day 7 from the initiation of the treatment of the present invention.

**Claims**

1. A method for activating lymphocyte in blood, comprising contacting blood with a carrier of a higher affinity to granulocyte than to lymphocyte, thereby removing granulocyte from the blood and subsequently adding cytokine to the blood in the state where the ratio of granulocyte to lymphocyte is decreased, whereby the lymphocyte in the blood is activated.

2. An apparatus for activating lymphocyte in blood, characterized by having a granulocyte adsorbing part containing a carrier with a higher affinity to granulocyte than to lymphocyte, and a cytokine injection part for adding cytokine to the blood flown out of the adsorbing part.

3. A therapeutical treatment of tumor, comprising contacting the blood flown out of a tumor patient to a carrier with a higher affinity to granulocyte than to lymphocyte to reduce the ratio of the number of granulocyte to the number of lymphocyte in the blood, subsequently adding cytokine to the blood thereby activating the lymphocyte in the blood and making the blood to return into the tumor patient.

4. The therapeutical treatment of tumor according to Claim 3, wherein the reduction of the ratio of granulocyte to lymphocyte and the addition of cytokine were carried out by using an apparatus for activating lymphocyte in blood, the apparatus being equipped with a granulocyte adsorbing part containing a carrier with a higher affinity to granulocyte than to lymphocyte, and a cytokine injection part to add cytokine to the blood flown out of the adsorbing part.

5. The therapeutical treatment of tumor according to Claim 3, wherein the flowing out of blood from a tumor patient and the returning of the blood containing activated lymphocyte are continuously carried out.

# Fig. 1

1. Granulocyte absorbing part

2. Carrier

3. Blood inflow part

4. Blood outflow part

5. Cytokine injection part

6. Cytokine injector

7. Circulation pump

8. Blood collecting part

9. Blood returning part

# Fig. 2

—△—: A group ( ≦2, 37 cases), 474 days (50% survival period)
—●—: B group (2<≦3, 37 cases), 311 days (50% survival period)
—○—: C group (3<≦4, 24 cases), 166 days (50% survival period)
—□—: D group ( 4<, 16 cases), 123 days (50% survival period)

generalized-wilcoxon test
A-B: P<0.05
B-C: P<0.01
C-D: P<0.05

Survival curve by each G/L ratio
of various progressive tumors (114 cases)   (Kaplan-Meier method)

EP 0 483 765 A2

# Fig. 3

○—○ : Circulation group    N=8
●—● : Control group      N=8
◉—◉ : Circulation + IL-2  N=2

Growth ratio (y-axis)

$$\text{Growth ratio} = \frac{\text{Proliferation size of tumor (multiplication of the major axis and the minor axis)}}{\text{Initial size of tumor (multiplication of the major axis and the minor axis)}}$$

Number of circulation (x-axis)